# EUROPEAN PATENT APPLICATION

(11) **EP 4 847 086 A1**
(43) Date of publication of application: **02.12.2026**
(21) Application number: 25745482.7
(22) Date of filing: 21.01.2025
(51) Int. Cl.: A61N 5/10

(54) **SYSTEM AND METHOD FOR CONTROLLING PULSED BEAM DEDICATED TO MULTI-GANTRY-STRUCTURED CARBON NANOTUBE X-RAY GENERATOR IN ORDER TO TREAT DEGENERATIVE BRAIN DISEASES**

(30) Priority: 24.01.2024 KR 20240010746; 09.12.2024 KR 20240181659
(71) Applicant: UNIVERSITY-INDUSTRY COOPERATION GROUP OF KYUNG HEE UNIVERSITY, Yongin-si, Gyeonggi-do 17104 (KR)
(72) Inventor: CHUNG, Weon Kuu, Seoul 05224 (KR)
(74) Representative: V.O.
(86) International application number: PCT/KR2025/001111
(87) International publication number: WO 2025/159465

(57) **Abstract**

The present invention relates to a pulse beam control system for treating a degenerative brain disease, and to a technology for precisely controlling irradiation intensity, a pulse width, a period, an irradiation angle, and the like of radiation to provide patient-customized radiation treatment. More specifically, the present invention includes a system capable of causing radiation to be concentrated on a lesion site through real-time dose monitoring and distance measurement, and minimizing radiation exposure of surrounding normal tissue.

## Description

### [Technical Field]

The present invention relates to a pulse beam control system for treating a degenerative brain disease, and more particularly, to a technology that provides an irradiation angle and energy optimized for a lesion site of a patient by using a multi-gantry structure, and maximizes safety and a treatment effect through real-time dose monitoring and feedback control.

### [Background Art]

1. This result was supported by the 2023 Hongneung Innopolis Specialized Growth Support Project [H-(Reverse) Train], conducted with the support of the Korea Innovation Foundation and funded by the Ministry of Science and ICT in 2023. "Research Project Title: Development of a Brain Drug Delivery System Using Carbon Nanotube-Based Low-Dose Radiation"
2. This result was supported by the Innopolis Specialized Growth Support Project (H-Bridge), conducted with the support of the Korea Innovation Foundation and funded by the Ministry of Science and ICT in 2023. "Research Project Title: Development of a Medical Device for Opening the Blood-Brain Barrier Using Carbon Nanotube-Based Low-Dose Ionizing Radiation"

Degenerative brain diseases, such as Alzheimer's disease and Parkinson's disease, are caused by neuroinflammation and pathological changes, and major treatment methods include drug therapy and non-invasive technologies. Recent studies have confirmed that low-dose radiation therapy (LDRT) is effective in suppressing neuroinflammation, reducing pathological markers, and protecting neurons, and efforts have been made to apply LDRT to treatment. When a continuous beam is used in low-dose radiation therapy, a constant radiation dose is continuously irradiated to provide treatment stability, but there are limitations in reducing tissue damage and precisely controlling biological responses. Accordingly, pulsed beam irradiation technology has attracted attention. Pulsed beam control technology is being developed as a technology capable of providing a high treatment effect to a lesion site while minimizing tissue damage by temporally controlling radiation through precise adjustment of a pulse width and a pulse period, and attempts are actively being made to improve the precision and efficiency of radiation therapy by introducing a multi-gantry structure capable of irradiating radiation at multiple angles and real-time dose control technology.

Since existing technologies cannot perform such control, treatment effects cannot be improved, and tissue damage and neuronal damage may be caused by unnecessary radiation exposure. If the pulse width and period of radiation irradiated according to a patient's condition cannot be precisely controlled, the treatment effect is reduced and the risk of side effects increases. In addition, due to insufficient real-time control functions, when an excessive dose or insufficient dose occurs during radiation irradiation, functions for immediately detecting and correcting such a situation are limited, and it is not possible to respond to changes in an irradiation angle and distance caused by movement or body shape changes of the patient.

A system to which pulsed beam irradiation technology is applied is attracting attention as a new approach for simultaneously securing the safety and effect of radiation therapy by strengthening precise control of radiation, real-time monitoring, and data integrated management functions in order to solve these problems.

### [Disclosure]

### [Technical Problem]

An object of the present invention is to maximize a treatment effect by measuring and adjusting a radiation dose for each patient in real time during radiation therapy.

Another object of the present invention is to use a multi-gantry structure so that radiation is uniformly irradiated to a lesion site at various angles.

Another object of the present invention is to provide customized treatment by optimizing radiation parameters, such as a number of pulses, a period, and energy intensity, according to individual characteristics of a patient.

Another object of the present invention is to systematically store and analyze data generated during radiation therapy and reflect the data in a subsequent treatment plan.

### [Technical Solution]

According to an embodiment, a pulse beam control system for treating a degenerative brain disease may include a pulse beam information setting unit configured to set pulse beam information including a number of pulses, a pulse period, a pulse beam irradiation angle, or pulse beam energy according to characteristics of a patient and a treatment purpose, a beam controller configured to individually control, in series or in parallel, at least one of a tube voltage, a tube current, a pulse width, an irradiation time, or an irradiation timing of a multi-port X-ray generator based on the set pulse beam information, a dose monitoring unit configured to monitor and store, in real time, patient-customized dose information corresponding to a generated pulse beam, and a data management unit configured to manage and output treatment data of the patient based on the monitored data.

According to an embodiment, the pulse beam information setting unit may automatically calculate and set the pulse beam information based on treatment planning data received from an external system.

According to an embodiment, the beam controller may provide an irradiation angle and an energy distribution optimized for a body part of the patient through a multi-gantry structure.

According to an embodiment, the dose monitoring unit may collect data by communicating with a Dose Rate Monitoring System and an SSD Monitoring System to measure real-time dose information of the patient.

According to an embodiment, the data management unit may analyze dose data collected from the dose monitoring unit, evaluate a treatment effect for each patient, and control output of the evaluated treatment effect.

According to an embodiment, the data management unit may store treatment data and dose data of the patient and construct a database so that the stored treatment data and dose data are reusable as data required for subsequent treatment.

According to an embodiment, the beam controller may synchronize irradiation timings of pulse beams generated by respective X-ray generators and irradiate the pulse beams in parallel or in series.

According to an embodiment, the dose monitoring unit may use a feedback control algorithm to optimize a dose and an energy intensity of an irradiated pulse beam for each patient.

According to an embodiment, a pulse beam control method for treating a degenerative brain disease may include setting a number of pulses, a pulse period, a pulse beam irradiation angle, and pulse beam energy according to characteristics of a patient and a treatment purpose, individually controlling, in series or in parallel, a tube voltage, a tube current, a pulse width, an irradiation time, and an irradiation timing of a multi-port X-ray generator based on the set information, monitoring and storing, in real time, patient-customized dose information corresponding to a controlled pulse beam, and analyzing and recording a treatment effect of the patient based on the stored data.

According to an embodiment, setting the number of pulses, the pulse period, the pulse beam irradiation angle, and the pulse beam energy may include automatically setting the number of pulses, the pulse period, the pulse beam irradiation angle, and the pulse beam energy based on patient data received from an external system.

According to an embodiment, controlling the multi-port X-ray generator may include providing an irradiation angle and an energy distribution optimized for a body part of the patient using a multi-gantry structure.

According to an embodiment, the pulse beam control method for treating a degenerative brain disease may further include adjusting a set dose and energy intensity by using a feedback control algorithm based on dose data measured in real time during a control process of the pulse beam.

### [Advantageous effects]

According to an embodiment, radiation exposure may be minimized and a treatment effect may be maximized through real-time dose monitoring and feedback control.

According to an embodiment, radiation may be uniformly distributed to a complex lesion structure by using a multi-gantry structure.

According to an embodiment, patient-customized radiation therapy may be provided through precise control of pulse beam parameters.

According to an embodiment, a long-term treatment effect may be analyzed and an improved treatment strategy may be established by using stored treatment data.

### [Description of Drawings]

FIG. 1 is a diagram illustrating a pulse beam control system for treating a degenerative brain disease according to an embodiment.
FIG. 2A is a diagram illustrating results indicating that there is no statistical significance in an Aβ plaque reduction effect when a continuous beam (KLDR) is applied.
FIG. 2B is a diagram illustrating results indicating that an Aβ plaque reduction effect is statistically significant when a pulse beam (PKLDR) is applied.
FIG. 3 is a diagram illustrating that, in a live imaging experiment, an expression level of M2-type microglia (CD206, anti-inflammatory action) is significantly increased when a pulse beam (PKLDR) is applied.
FIG. 4 is a diagram illustrating that, in a live imaging experiment, expression levels of Aβ42 and tau, which are disease markers, are significantly decreased when a pulse beam (PKLDR) is applied.
FIG. 5 is a diagram indicating that inflammatory markers (TNF-α and IL-1β) are significantly decreased, while a recovery gene (RSPO1), and activity markers of dopamine neurons (VMAT2 and DAT) are increased when a pulse beam (PKLDR) is applied.
FIG. 6 is a diagram illustrating a pulse beam precisely controlled by the present invention.
FIG. 7 is a diagram illustrating application fields of the present invention.
FIG. 8 is a diagram illustrating an operation system diagram according to an embodiment.
FIG. 9 is a diagram illustrating an operation method of a pulse beam control system for treating a degenerative brain disease according to an embodiment.

### [Best Mode]

Specific structural or functional descriptions of embodiments according to the concept of the present invention disclosed in the present specification are merely illustrated for the purpose of describing the embodiments according to the concept of the present invention, and the embodiments according to the concept of the present invention may be embodied in various forms and are not limited to the embodiments described in the present specification.

Since the embodiments according to the concept of the present invention may have various modifications and various forms, the embodiments are illustrated in the drawings and will be described in detail in the present specification. However, this is not intended to limit the embodiments according to the concept of the present invention to specific disclosed forms, and should be understood to include modifications, equivalents, or substitutes included in the spirit and technical scope of the present invention.

Terms such as first or second may be used to describe various components, but the components should not be limited by the terms. The terms are used only for the purpose of distinguishing one component from another component. For example, without departing from the scope according to the concept of the present invention, a first component may be referred to as a second component, and similarly, a second component may also be referred to as a first component.

When a component is referred to as being "connected" or "coupled" to another component, it should be understood that the component may be directly connected or coupled to the other component, or another component may be present therebetween. On the other hand, when a component is referred to as being "directly connected" or "directly coupled" to another component, it should be understood that no other component is present therebetween. Expressions describing relationships between components, such as "between" and "directly between" or "directly adjacent to," should be interpreted in the same manner.

The terms used in the present specification are used only to describe specific embodiments and are not intended to limit the present invention. Singular expressions include plural expressions unless the context clearly indicates otherwise. In the present specification, terms such as "comprise" or "have" are intended to indicate the presence of stated features, numbers, steps, operations, components, parts, or combinations thereof, and should be understood not to preclude in advance the presence or addition of one or more other features, numbers, steps, operations, components, parts, or combinations thereof.

Unless otherwise defined, all terms used herein, including technical or scientific terms, have the same meanings as generally understood by a person having ordinary skill in the art to which the present invention pertains. Terms such as those defined in commonly used dictionaries should be interpreted as having meanings consistent with their meanings in the context of the related art, and should not be interpreted in an idealized or excessively formal sense unless expressly defined in the present specification.

Hereinafter, embodiments will be described in detail with reference to the accompanying drawings. However, the scope of the patent application is not limited or restricted by these embodiments. Like reference numerals shown in the respective drawings indicate like members.

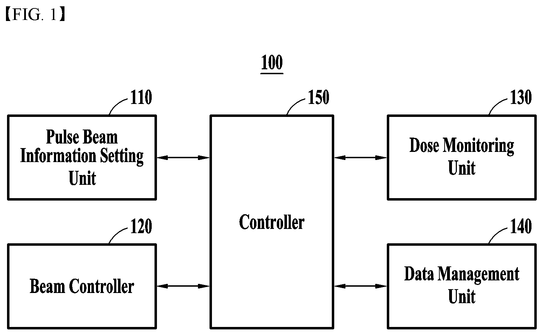

FIG. 1 is a diagram illustrating a pulse beam control system 100 for treating a degenerative brain disease according to an embodiment.

FIG. 1 is a block diagram showing components of the pulse beam control system 100 for treating a degenerative brain disease and interactions therebetween, and the pulse beam control system 100 integrally performs setting, control, monitoring, and data management of a pulse beam for patient-customized treatment. All components are organically connected and operated around a central controller 150, and roles and detailed functions of the respective components are as follows.

A pulse beam information setting unit 110 sets pulse beam information according to a treatment plan and a purpose of a patient.

The pulse beam information setting unit 110 according to an embodiment sets pulse beam information including a number of pulses, a pulse period, a pulse beam irradiation angle, or pulse beam energy according to characteristics of the patient and a treatment purpose. The number of pulses is increased when stimulation is required according to characteristics of a treatment site, or is decreased for sensitive tissue to minimize side effects.

The pulse period promotes tissue recovery with a long period or provides a continuous stimulation effect with a short period. The pulse beam irradiation angle irradiates radiation in a direction optimized for a treatment target site, and, for example, precisely irradiates radiation to a specific brain region to maximize a radiation effect.

The pulse beam energy is set such that high energy is suitable for deep tissue treatment and low energy is suitable for surface treatment. The pulse beam information setting unit 110 according to an embodiment automatically calculates the pulse beam information through treatment planning data received from an external system and generates an optimal setting value based on MRI data of the patient.

A beam controller 120 according to an embodiment individually controls, in series or in parallel, at least one of a tube voltage, a tube current, a pulse width, an irradiation time, or an irradiation timing of a multi-port X-ray generator based on the set pulse beam information. In particular, the beam controller 120 adjusts parameters of the X-ray generator based on the set pulse beam information.

The tube voltage determines energy of a beam, and a high voltage is used for deep tissue and a low voltage is used for surface treatment.

The tube current controls an amount of the beam, and the pulse width adjusts a total radiation exposure amount. Synchronization of the irradiation timing allows radiation to be irradiated from a plurality of ports in parallel or in series, and a multi-gantry structure provides an irradiation angle and an energy distribution optimized according to a body structure and a lesion location of the patient.

The beam controller 120 according to an embodiment precisely controls radiation energy and a direction for patient-customized treatment, and operates in association with a controller 150 in real time.

The beam controller 120 according to an embodiment may provide an irradiation angle and an energy distribution optimized for a body part of a patient through a multi-gantry structure.

The beam controller 120 according to an embodiment may synchronize irradiation timings of pulse beams generated by respective X-ray generators and irradiate the pulse beams in parallel or in series.

The dose monitoring unit 130 according to an embodiment monitors and stores, in real time, patient-customized dose information corresponding to a generated pulse beam. In addition, the dose monitoring unit 130 may collect data by communicating with a Dose Rate Monitoring System and an SSD Monitoring System to measure real-time dose information of the patient.

The dose monitoring unit 130 according to an embodiment monitors and analyzes, in real time, dose data delivered to the patient during radiation irradiation. The Dose Rate Monitoring System measures an instantaneous rate of a radiation dose, and the SSD Monitoring System measures a distance between an X-ray generator and the patient to maintain accuracy of a beam.

The measured data is transmitted to the data management unit 140, which manages and outputs treatment data of the patient based on the data. In particular, the data management unit 140 analyzes dose data collected from the dose monitoring unit, evaluates a treatment effect for each patient, and controls output of the evaluated treatment effect. The dose monitoring unit 130 according to an embodiment ensures safety of treatment and operates integrally with the controller 150. In addition, the data management unit 140 stores treatment data and dose data of the patient and constructs a database so that the stored treatment data and dose data are reusable as data required for subsequent treatment.

The dose monitoring unit 130 according to an embodiment may use a feedback control algorithm to optimize a dose and an energy intensity of an irradiated pulse beam for each patient.

In particular, the dose monitoring unit 130 collects data by communicating with a Dose Rate Monitoring System and an SSD Monitoring System to measure real-time dose information of the patient. The dose monitoring unit 130 uses a feedback control algorithm to optimize a dose and an energy intensity of an irradiated pulse beam for each patient.

The data management unit 140 according to an embodiment collects, stores, analyzes, and outputs data generated by the system.

The data management unit 140 according to an embodiment may analyze dose data collected from the dose monitoring unit, evaluate a treatment effect for each patient, and control output of the evaluated treatment effect.

In addition, the data management unit 140 according to an embodiment may store treatment data and dose data of the patient and construct a database so that the stored treatment data and dose data are reusable as data required for subsequent treatment.

Data received from the dose monitoring unit 130 according to an embodiment is analyzed to evaluate a treatment effect, such as a lesion size reduction rate and a patient recovery speed. All data is stored in the database and used for subsequent treatment and research. Treatment data is visually output and provided to medical staff and the patient, thereby improving reliability. The data management unit is used as material for implementing precision medicine.

The controller 150 according to an embodiment is a central processing module that integrally manages data among the pulse beam information setting unit 110, the beam controller 120, the dose monitoring unit 130, and the data management unit 140 and transmits commands.

The controller 150 according to an embodiment maintains stability of the system by coordinating data flow among the respective components.

The controller 150 detects abnormal data flow and immediately responds thereto to ensure stability. The controller 150 according to an embodiment coordinates the respective components to achieve optimal performance, and is responsible for overall data flow and commands.

The pulse beam control system 100 for treating a degenerative brain disease according to an embodiment starts when the pulse beam information setting unit 110 sets pulse beam information according to a treatment plan of the patient.

The pulse beam information setting unit 110 automatically calculates and sets the pulse beam information based on treatment planning data received from an external system.

The controller 150 transmits this information to the beam controller 120 to control the X-ray generator. The beam controller 120 adjusts physical characteristics of a beam based on the set information and performs treatment. The dose monitoring unit 130 measures, in real time, a dose and energy of the irradiated beam and transmits data to the controller 150.

The beam controller 120 provides an irradiation angle and an energy distribution optimized for a body part of the patient through a multi-gantry structure. In addition, the beam controller 120 synchronizes irradiation timings of pulse beams generated by respective X-ray generators and irradiates the pulse beams in parallel or in series.

The data management unit 140 stores collected data to evaluate a treatment effect and uses the collected data for subsequent treatment. The pulse beam control system 100 for treating a degenerative brain disease according to an embodiment supports patient-customized precision treatment of a degenerative brain disease, and is designed to maximize a treatment effect and safety.

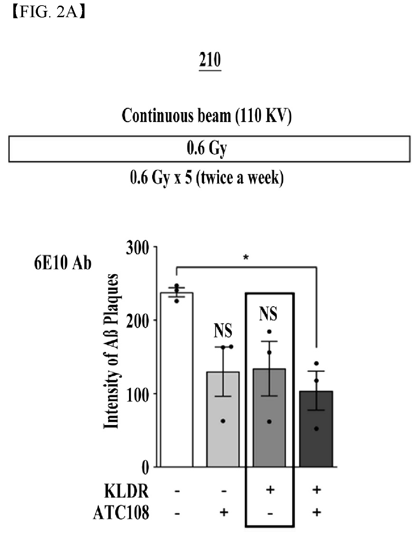

FIG. 2A is a diagram 210 illustrating results indicating that there is no statistical significance in an Aβ plaque reduction effect when a continuous beam (KLDR) is applied.

FIG. 2A indicates that, as a result of applying a continuous beam (KLDR, Kilovoltage Low-Dose Radiation) to an Alzheimer's disease animal model, there is no statistical significance in a reduction effect of Aβ plaques (Intensity of Aβ Plaques).

The X-axis indicates four experimental groups, and the respective groups follow the following treatment conditions.

A first group is a control group in which KLDR is not applied (-) and ATC108 is not applied (-), and thus both radiation irradiation and drug administration are not performed. This is based on a state in which treatment is not performed in the Alzheimer's disease animal model.

A second group is a group in which KLDR is applied (+) and ATC108 is not applied (-), and a 110 kV continuous beam (KLDR) is applied but the drug ATC108 is not administered. This is a condition for evaluating an effect of radiation alone.

A third group is a group in which KLDR is not applied (-) and ATC108 is applied (+), and radiation irradiation is not performed but the drug ATC108 is administered alone. This is a condition for confirming an effect of the drug alone.

A fourth group is a group in which KLDR is applied (+) and ATC108 is applied (+), and the continuous beam (KLDR) and the drug ATC108 are applied in combination. This is a condition for evaluating an interaction and a combined effect of the radiation and the drug.

The Y-axis quantitatively represents an intensity of Aβ plaques. This reflects a relative concentration of Aβ plaques in brain tissue and is an important indicator capable of indirectly evaluating a progression degree of Alzheimer's disease. Each bar graph represents an average value of an experimental group, and an error bar represents a standard error of mean (SEM) showing variability of data.

The radiation used is a 110 kV continuous beam (KLDR), and radiation intensity is set to 0.6 Gy. Radiation irradiation is performed twice a week for a total of five times, and thus a cumulative radiation dose corresponds to 3 Gy.

The continuous beam (KLDR) has constant radiation intensity and duration, and, unlike a pulsed beam (PKLDR) method, continuously irradiates energy without interruption. The drug ATC108 is a new drug candidate developed for treating Alzheimer's disease, and is expected to have an Aβ plaque reduction effect and an anti-inflammatory effect. A concentration and a dose of the administered drug are maintained constant according to an experimental design.

The highest Aβ plaque intensity is observed in the control group to which neither the continuous beam nor the drug is applied. This reflects a result in which Alzheimer's disease has progressed in a state in which treatment is not performed.

In the KLDR-alone group, as a result of applying the continuous beam alone, the Aβ plaque intensity is lower than that of the control group, but statistical significance (NS, Not Significant) is not secured.

This suggests that application of the continuous beam alone provides a limited effect on reduction of Aβ plaques. Also in the group in which only the drug ATC108 is administered alone, the Aβ plaque intensity is reduced, but statistical significance (NS) is not secured. This shows that the drug-alone effect is limited.

In the group in which KLDR and ATC108 are applied in combination, the Aβ plaque intensity is lowest, but statistical significance is still not secured. Although the combined effect induces a greater reduction than the single effect, it is insufficient to prove a substantial treatment effect.

FIG. 2A illustrates results obtained by quantitatively evaluating effects of radiation-alone application, drug-alone application, and combined application of the KLDR method on reduction of Aβ plaques in the Alzheimer's disease animal model.

As a result of the experiment, the continuous beam (KLDR) induces reduction of Aβ plaques, but does not secure statistical significance, suggesting that a treatment effect is limited. This shows that the continuous beam method alone is not sufficient for treating Alzheimer's disease, and that a more effective treatment method is needed. This experiment shows a limitation of the continuous beam method and supports the possibility that a pulsed beam (PKLDR) may be more effective. According to existing research, the pulsed beam showed superior results not only in reduction of Aβ plaques but also in anti-inflammatory effects and improvement of disease markers. Therefore, the present experimental results emphasize the need to shift to the pulsed beam method. As future research, it is necessary to more clearly evaluate treatment effects of the two methods through a direct comparison experiment between KLDR and PKLDR. In addition, it is necessary to adjust a concentration and a dose of the drug ATC108 to explore optimal conditions for drug-alone application or combined application.

KLDR means continuous beam (Continuous Kilovoltage Low-Dose Radiation), and ATC108 is a drug candidate material for treating Alzheimer's disease. Aβ plaques indicate an intensity of beta-amyloid plaques, which are a pathological feature of Alzheimer's disease, and NS (Not Significant) means that there is no statistical significance. The error bars show a standard error of data of each experimental group. These results clearly show that the treatment effect of the continuous beam (KLDR) is limited, and emphasize that a more effective approach is needed to reduce Aβ plaques.

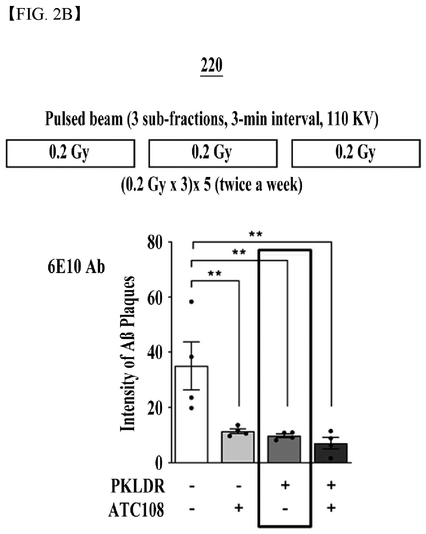

FIG. 2B is a diagram 210 illustrating results indicating that an Aβ plaque reduction effect is statistically significant when a pulsed beam (PKLDR) is applied.

FIG. 2B is a diagram showing that a pulsed beam (PKLDR, Pulsed Kilovoltage Low-Dose Radiation) exhibits an Aβ plaque (Intensity of Aβ Plaques) reduction effect in an Alzheimer's disease animal model, and proves a statistically significant effect.

This experiment is designed to evaluate effects of radiation-alone application, drug-alone application, and combined application of the PKLDR method on reduction of Aβ plaques, which are a pathological feature of Alzheimer's disease. The results prove superiority of PKLDR over an existing continuous beam (KLDR), and also quantitatively confirm a combined effect with the drug ATC108.

The X-axis of FIG. 2B is configured by dividing experimental groups into four groups.

A first group is a control group in which PKLDR is not applied (-) and ATC108 is not applied (-), and thus neither radiation nor the drug is applied. This represents a result in which Alzheimer's disease has progressed in a state in which treatment is not performed.

A second group is a group in which PKLDR is applied (+) and ATC108 is not applied (-), and the pulsed beam (PKLDR) is applied alone. This group is important in confirming the possibility that the PKLDR method improves a treatment effect over the existing continuous beam (KLDR).

A third group is a group in which PKLDR is not applied (-) and ATC108 is applied (+), and only the drug ATC108 is administered alone. This is a condition for evaluating an independent treatment effect of the drug.

A fourth group is a group in which PKLDR is applied (+) and ATC108 is applied (+), and PKLDR and the drug ATC108 are applied in combination. This is a condition for evaluating an interaction and a combined effect of the radiation and the drug.

The Y-axis quantitatively represents an intensity of Aβ plaques (Intensity of Aβ Plaques), which indicates a progression degree of Alzheimer's disease. The plaque intensity reflects a relative concentration of beta-amyloid plaques in brain tissue, which is a major indicator of a pathological condition of Alzheimer's disease.

The bar graph represents an average value of each experimental group, and the error bar represents a standard error showing variability of data. In the drawing, statistical significance is indicated by an asterisk (), and indicates a significant difference at a level of p < 0.01. This improves reliability of the experimental results.

The pulsed beam (PKLDR) is set to 110 kV energy, and 0.2 Gy is applied in each irradiation. Radiation irradiation is performed by dividing the irradiation into three sub-fractions, and an interval between irradiations is set to 3 minutes.

Such pulsed beam irradiation is performed twice a week for a total of five times, and a total cumulative radiation dose corresponds to 3 Gy. The PKLDR method is characterized in that tissue damage is minimized and a radiation effect is maximized by irradiating radiation in divided pulses at short intervals.

This is a main difference from the continuous beam (KLDR). ATC108 is a drug candidate developed for treating Alzheimer's disease, and aims at reduction of Aβ plaques and an anti-inflammatory effect. The drug is administered at a constant concentration and dose according to an experimental design, and may maximize a plaque reduction effect when combined with radiation.

The control group to which neither radiation nor the drug is applied exhibits the highest Aβ plaque intensity. This reflects that a pathological feature of Alzheimer's disease is maintained as it is in a state in which treatment is not performed.

In a group to which the pulsed beam (PKLDR) is applied alone, the Aβ plaque intensity is significantly reduced compared with that of the control group (p < 0.01). This suggests that the PKLDR method may improve a radiation treatment effect over the continuous beam (KLDR), and shows that plaque reduction may be induced while minimizing tissue damage.

Also in a group to which the drug ATC108 is administered alone, the Aβ plaque intensity is significantly reduced compared with that of the control group (p < 0.01). This indicates that the drug has a potential treatment effect capable of independently inducing Aβ plaque reduction even without radiation.

In a group to which PKLDR and ATC108 are applied in combination, the Aβ plaque intensity is lowest among all experimental groups. The combined application exhibits a greater treatment effect than the single application, and exhibits a statistically highly significant reduction (p < 0.01) in plaque reduction.

This indicates that PKLDR and the drug exhibit complementary effects, and that combined treatment presents an important possibility in treating Alzheimer's disease.

FIG. 2B visually illustrates results obtained by quantitatively evaluating effects of radiation-alone application, drug-alone application, and combined application of the PKLDR method on reduction of Aβ plaques.

Experimental results show that the pulsed beam (PKLDR) exhibits a more superior Aβ plaque reduction effect than the continuous beam (KLDR), and exhibits statistically significant results. This suggests that tissue damage may be reduced and a treatment effect may be maximized by dividing radiation irradiation. ATC108 may induce Aβ plaque reduction even without radiation, and plays an important role in pathological improvement. Combined treatment of the pulsed beam and the drug exhibits a greater effect in plaque reduction than a single treatment, which shows that an interaction between radiation and the drug is a promising strategy in treating Alzheimer's disease.

PKLDR means Pulsed Kilovoltage Low-Dose Radiation, and is a pulsed beam method that minimizes tissue damage and maximizes a radiation effect. ATC108 is a drug candidate for treating Alzheimer's disease, and is expected to exhibit an Aβ plaque reduction effect and an anti-inflammatory effect.

Aβ plaques are beta-amyloid plaques, which are a major pathological feature of Alzheimer's disease. p < 0.01 indicates a statistically highly significant result. The error bar is a standard error indicating variability of data. These results prove that PKLDR is a superior treatment method for treating Alzheimer's disease over the existing KLDR method, and emphasize a treatment synergy effect through combination with the drug.

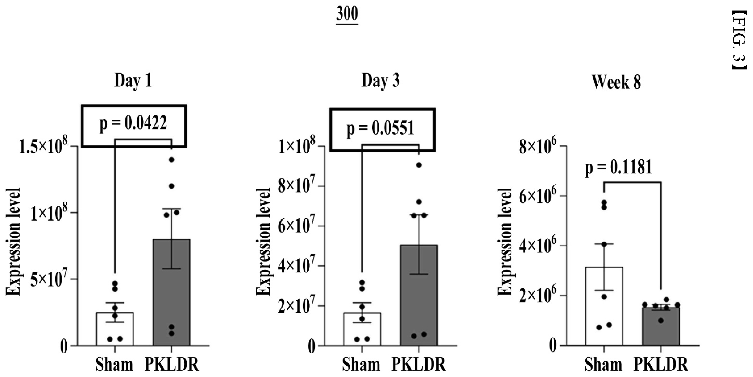

FIG. 3 is a diagram 300 illustrating that, in a live imaging experiment, an expression level of M2-type microglia (CD206, anti-inflammatory action) is significantly increased when a pulsed beam (PKLDR) is applied.

FIG. 3 illustrates results obtained by measuring a change in an expression level of M2-type microglia (CD206) in a live imaging experiment to which a pulsed beam (PKLDR, Pulsed Kilovoltage Low-Dose Radiation) is applied, thereby evaluating inflammatory response regulation and an anti-inflammatory effect.

M2-type microglia are cells that suppress inflammation and promote tissue repair, and visually show an effect of application of PKLDR on expression of the cells. The experiment is designed to analyze an effect of PKLDR on regulation of an inflammatory environment and to track changes over time.

In FIG. 3, differences in expression levels between a control group (Sham) and a PKLDR-applied group are compared at three time points of Day 1, Day 3, and Week 8.

The X-axis is divided into Sham and PKLDR. Sham is a group to which radiation is not irradiated, and represents a natural level of expression of M2-type microglia.

PKLDR is a group to which the pulsed beam (PKLDR) is applied, and evaluates a change in expression of M2-type microglia and an anti-inflammatory effect after radiation irradiation. The Y-axis quantitatively represents an expression level of M2-type microglia (CD206), and a unit thereof is an expression level.

The expression level reflects a degree of cell activation, and is an important indicator capable of evaluating regulation of inflammation and intensity of an anti-inflammatory response.

FIG. 3 compares results at three time points.

Day 1 is a result on a first day after radiation irradiation, and evaluates an initial effect. Day 3 is a result on a third day after radiation irradiation, and confirms persistence of an initial anti-inflammatory effect. Week 8 is a result at eight weeks after radiation irradiation, and evaluates a long-term effect.

The bar graph represents an average value of each experimental group, and the error bar represents a standard error showing variability of data. Statistical significance is indicated by a p-value, and p < 0.05 indicates a significant difference and is used to verify an effect of PKLDR.

Under the PKLDR application condition, radiation energy is set to 110 kV, and radiation is irradiated by being divided into three sub-fractions of 0.2 Gy.

An interval between respective irradiations is set to 3 minutes, and irradiation is performed twice a week for a total of five times. The PKLDR method divides and irradiates radiation in a pulsed form to induce effective inflammation regulation while minimizing tissue damage. Under the Sham condition, radiation is not irradiated, and the Sham condition is set as a control group for evaluating a natural state of the expression level of M2-type microglia.

On Day 1, the expression level of M2-type microglia in the PKLDR group is significantly increased compared with that of the control group (Sham) (p = 0.0422). This indicates that PKLDR regulates an inflammatory environment and induces an initial anti-inflammatory response. A rapid increase in the expression level is interpreted as a result of radiation stimulating M2-type microglia to be activated and converted into an anti-inflammatory state. In contrast, in the Sham group, the expression level is maintained at a low level, because there is no radiation or external stimulation.

On Day 3, the expression level of M2-type microglia in the PKLDR group is somewhat increased compared with Day 1, but a difference from the control group does not reach statistical significance, with p = 0.0551.

This suggests that the initial effect is being maintained, but shows a possibility that natural healing or a change in an inflammatory environment contributes to a decrease in the effect. In the Sham group, the expression level is still maintained at a low level, and a difference from the PKLDR group tends to decrease.

At Week 8, the expression level of the PKLDR group is decreased, and a difference from the control group is reduced, and statistical significance is not secured, with p = 0.1181. This indicates that the anti-inflammatory effect of PKLDR decreases in the long term, or that the difference in the expression level decreases due to a natural change in an immune environment. In the Sham group, the expression level is maintained at a level similar to the initial level.

PKLDR induces an initial anti-inflammatory effect and significantly increases the expression level of M2-type microglia at Day 1. This proves that radiation is effective in regulating an inflammatory environment and promoting an anti-inflammatory state. At Day 3, the expression level of the PKLDR group is maintained, but a difference from the control group tends to decrease. This suggests that PKLDR maintains an initial effect, but may be affected by a change in an inflammatory environment or a natural healing process over time.

At Week 8, the effect of PKLDR decreases, and a difference from the control group is not significant. This suggests that additional radiation therapy or a combined treatment strategy may be required to maximize the effect of PKLDR in the long term.

PKLDR is a method that divides and irradiates radiation at short intervals to minimize tissue damage and maximize an effect of regulating an inflammatory response. M2-type microglia (CD206) are anti-inflammatory microglia that suppress inflammation and promote tissue repair, and play an important role in regulating an inflammatory environment.

Sham is a control group to which radiation is not irradiated, and represents a natural expression state of M2-type microglia. A p-value indicates statistical significance, and p < 0.05 indicates a significant difference. The error bar means a standard error indicating variability of data.

PKLDR is effective in regulating an initial inflammatory response, and significantly increases the expression level of M2-type microglia at Day 1.

This suggests that the PKLDR method is a promising method for inducing inflammation suppression and anti-inflammatory activation in treating a degenerative brain disease. However, a tendency that the effect gradually decreases at Day 3 and Week 8 is observed, which shows that additional strategies, such as repeated PKLDR application or drug combined treatment, are required to maximize a long-term effect.

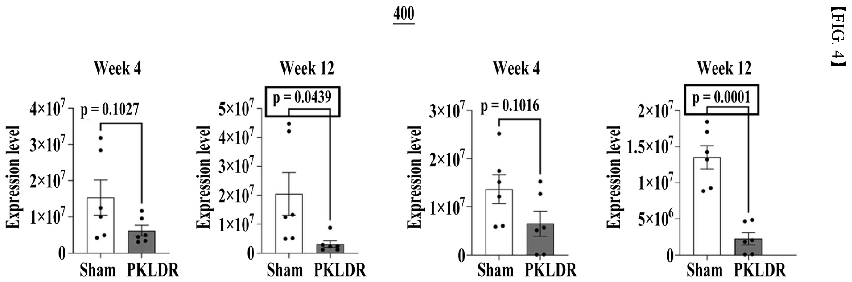

FIG. 4 is a diagram 400 illustrating that, in a live imaging experiment, expression levels of Aβ42 and tau, which are disease markers, are significantly decreased when a pulsed beam (PKLDR) is applied.

FIG. 4 is a diagram illustrating results obtained by evaluating an effect of a pulsed beam (PKLDR, Pulsed Kilovoltage Low-Dose Radiation) on reduction of expression levels of Aβ42 and tau proteins, which are disease markers, in an Alzheimer's disease animal model over time.

Aβ42 and tau are key indicators reflecting pathological progression of Alzheimer's disease, and reduction of expression levels of these materials is regarded as an important measure for evaluating disease suppression and a treatment effect.

The experiment of FIG. 4 is designed to analyze how the PKLDR method changes expression of these pathological markers, and FIG. 4 compares differences in expression levels between a control group (Sham) and a PKLDR-applied group at two time points of Week 4 and Week 12, thereby verifying short-term and long-term effects of PKLDR.

The X-axis indicates two experimental conditions of Sham and PKLDR, and shows expression level results at Week 4 and Week 12 for each of Aβ42 and tau proteins.

Sham is a group to which radiation is not irradiated, and is a reference group for evaluating natural expression states of Aβ42 and tau. PKLDR is a group for evaluating an effect of radiation irradiation on expression levels of disease markers. The Y-axis quantitatively represents expression levels of Aβ42 and tau, and a unit thereof is an expression level. The expression levels directly reflect a pathological state, and reduction of these materials indicates suppression of disease progression and a treatment effect.

The bar graph represents an average value of each experimental group, and the error bar shows a standard error indicating variability of data. Statistical significance is indicated by a p-value, and p < 0.05 indicates a significant difference. Aβ42 shows a significant reduction at Week 12, with p = 0.0439, and tau shows a statistically highly significant reduction at Week 12, with p = 0.0001.

In PKLDR, radiation energy is set to 110 kV, and radiation is irradiated by being divided into three sub-fractions of 0.2 Gy. An interval between respective irradiations is set to 3 minutes, and irradiation is performed twice a week for a total of five times.

PKLDR minimizes tissue damage and maximizes a treatment effect by dividing and irradiating radiation in a pulsed form. The Sham group is set as a control group to which radiation is not irradiated, and is used to compare and analyze a treatment effect of PKLDR based on natural expression states of Aβ42 and tau.

At Week 4, the expression level of Aβ42 in the PKLDR group is decreased compared with that of the control group, but statistical significance is not secured, with p = 0.1027. In the case of tau, the expression level in the PKLDR group is also somewhat decreased compared with that of the control group, but statistical significance is not secured, with p = 0.1016. This indicates that an initial effect of PKLDR is not clear at Week 4.

At Week 12, the expression level of Aβ42 in the PKLDR group is significantly decreased compared with that of the control group, and statistical significance is secured, with p = 0.0439. In the case of tau, the expression level in the PKLDR group is significantly decreased compared with that of the control group, and shows a statistically highly significant difference, with p = 0.0001. This indicates that PKLDR is effective in suppressing expression levels of Aβ42 and tau proteins in the long term.

PKLDR significantly decreases expression levels of Aβ42 and tau, which are major pathological indicators of Alzheimer's disease, at Week 12. This proves that PKLDR is an effective approach for suppressing progression of Alzheimer's disease and treating the disease.

At Week 4, the effect of PKLDR is not clear, but at Week 12, a significant reduction in expression levels is observed. This suggests that PKLDR requires a certain period of time to exert a treatment effect and may exhibit a greater effect from a long-term perspective. PKLDR has a superior disease marker suppression effect compared with an existing continuous beam (KLDR) method, and is evaluated as a promising radiation technology in treating Alzheimer's disease.

PKLDR is a pulsed low-dose radiation technology that maximizes a treatment effect while minimizing tissue damage. Aβ42 and tau are major disease markers reflecting pathological progression of Alzheimer's disease, and reduction of expression levels thereof indicates a disease suppression effect.

Sham is a control group to which radiation is not irradiated, and is a reference group for evaluating natural states of Aβ42 and tau expression. A p-value indicates statistical significance, and p < 0.05 indicates a significant difference. The error bar is a standard error indicating variability of data, and improves reliability of each experimental group.

PKLDR significantly decreases expression levels of Aβ42 and tau, which are disease markers, and proves that PKLDR is effective in suppressing progression of Alzheimer's disease. In particular, statistically significant results at Week 12 support a long-term treatment possibility of PKLDR.

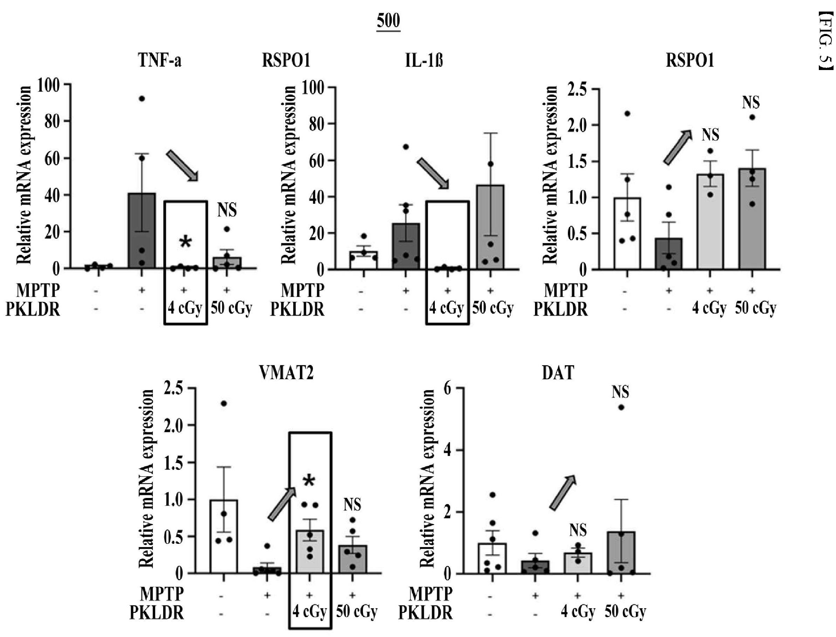

FIG. 5 is a diagram 500 indicating that inflammatory markers (TNF-α and IL-1β) are significantly decreased, while a recovery gene (RSPO1), and activity markers of dopamine neurons (VMAT2 and DAT) are increased when a pulse beam (PKLDR) is applied.

FIG. 5 is a diagram illustrating results obtained by quantitatively evaluating effects of a pulsed beam (PKLDR, Pulsed Kilovoltage Low-Dose Radiation) on changes in expression of neuroinflammatory markers (TNF-α and IL-1β), a recovery gene (RSPO1), and dopamine pathway proteins (VMAT2 and DAT).

The experiment of FIG. 5 is designed to confirm whether PKLDR is effective in suppressing an inflammatory response and activating neural recovery and a dopamine pathway by using a Parkinson's disease animal model (an MPTP-induced model).

TNF-α and IL-1β are major markers causing neuroinflammation, and reduction in expression thereof indicates alleviation of inflammation and suppression of tissue damage.

RSPO1 is an important recovery gene involved in tissue repair and regeneration, and VMAT2 and DAT are major proteins responsible for dopamine storage and transport, and increase in expression thereof means recovery of dopamine pathway function.

FIG. 5 quantitatively evaluates an effect of PKLDR by comparing differences in expression levels among two radiation dose conditions of PKLDR (4 cGy and 50 cGy) and a control group (MPTP).

The X-axis indicates experimental conditions, and the control group (MPTP) is a group in which dopamine neuron damage is induced, and effects of PKLDR are compared based on natural expression levels of the respective markers.

PKLDR 4 cGy is a group for analyzing effects of radiation on an inflammatory response and expression of a recovery gene under a low-dose condition, and PKLDR 50 cGy is a group for evaluating an effect under a high-dose condition.

The Y-axis quantitatively represents relative mRNA expression levels of the respective markers (TNF-α, IL-1β, RSPO1, VMAT2, and DAT), and represents a rate of increase or decrease relative to a reference group. The expression levels are important indicators for evaluating suppression of inflammation, tissue repair, and activation of a dopamine pathway.

The bar graph represents an average value of each experimental group, and the error bar shows a standard error. Statistical significance is indicated by a p-value, and p < 0.05 indicates a significant difference. In FIG. 5, an asterisk (*) indicates a significant result, and "NS" indicates a result that is not statistically significant.

Under the PKLDR condition, radiation energy is set to 110 kV, and radiation intensity is applied under two conditions of 4 cGy and 50 cGy.

Radiation is irradiated in a short pulsed form, and an interval between respective pulse irradiations is set to 3 minutes.

Irradiation is performed twice a week for a total of five times. PKLDR is a radiation technology capable of maximizing an anti-inflammatory effect while minimizing tissue damage. The control group (MPTP) is set as a reference group for evaluating a treatment effect of PKLDR, by inducing dopamine neuron damage and causing neuroinflammation and functional impairment.

The expression level of TNF-α is significantly decreased compared with the control group under the PKLDR 4 cGy condition (p < 0.05). This indicates that low-dose PKLDR exerts an anti-inflammatory effect by suppressing a major inflammatory substance.

Under the PKLDR 50 cGy condition, a decrease in the expression level is observed, but is not statistically significant. The expression level of IL-1β shows a decreasing tendency under both conditions, but there is no statistically significant difference. This suggests that an effect of PKLDR on suppression of IL-1β is relatively insignificant.

The expression level of RSPO1 shows an increasing tendency under the PKLDR 4 cGy condition, but is not statistically significant. This suggests that low-dose PKLDR may promote neural tissue repair. Under the PKLDR 50 cGy condition, an increase in the expression level is also observed, but is not statistically significant.

The expression level of VMAT2 is significantly increased compared with the control group under the PKLDR 4 cGy condition (p < 0.05). This indicates that low-dose PKLDR may promote recovery of neuron function related to dopamine storage.

Under the PKLDR 50 cGy condition, an increase in the expression level is observed, but is not statistically significant. The expression level of DAT shows an increasing tendency under both conditions, but there is no statistically significant difference. This suggests that an effect of PKLDR on activation of a dopamine transport pathway may be limited.

PKLDR 4 cGy proves to be effective in suppressing a neuroinflammatory response by significantly decreasing TNF-α expression. This presents a possibility that PKLDR may become an important treatment approach in neuroinflammatory diseases such as Parkinson's disease. The increasing tendency of the expression level of RSPO1 indicates that PKLDR may promote neural tissue repair and regeneration.

Although statistical significance is not secured, activation of a recovery gene is regarded as an important indicator for evaluating a long-term treatment effect. Through an increase in the expression level of VMAT2, it is confirmed that PKLDR 4 cGy may promote recovery of neuron function related to dopamine storage. This supports a treatment possibility of PKLDR in Parkinson's disease related to dopamine deficiency.

PKLDR proves to be effective in suppressing neuroinflammation and recovering neuron function by suppressing a major inflammatory marker such as TNF-α and significantly increasing expression of a dopamine pathway protein such as VMAT2.

The increasing tendencies of expression levels of RSPO1 and DAT suggest that PKLDR may play a positive role in neural tissue repair and activation of a dopamine pathway. PKLDR technology is evaluated as an innovative treatment approach that simultaneously targets inflammation suppression and neural recovery, and may play an important role in treating degenerative neurological diseases.

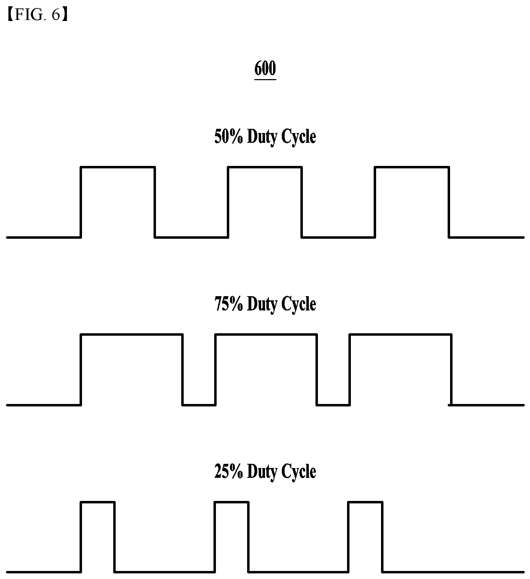

FIG. 6 is a diagram 600 illustrating a pulse beam precisely controlled by the present invention.

FIG. 6 is a diagram visually illustrating various duty cycle forms of a pulse beam precisely controlled based on a pulsed beam (PKLDR, Pulsed Kilovoltage Low-Dose Radiation) technology.

FIG. 6 shows a process of implementing optimized radiation irradiation according to a treatment purpose and individual characteristics of a patient by changing a pulse width and a ratio of an irradiation time through duty cycle control, which is a core technology of a pulse beam control system.

A duty cycle is defined as a ratio between an "On" time of a pulse and an entire period, and plays an important role in controlling a biological stimulation effect and a tissue response of the pulse beam.

FIG. 6 includes three duty cycle examples of 50%, 75%, and 25%, and specifically shows changes in a pulse width and an irradiation ratio according to each duty cycle.

In the 50% duty cycle, an "On" state and an "Off" state of the pulse beam are set at the same ratio, and radiation is irradiated only for half of an entire irradiation time.

This is suitable for maintaining a balance between a biological stimulation effect and tissue damage, and may be used to secure safety and efficiency in an initial treatment stage of Alzheimer's disease or Parkinson's disease.

In the 75% duty cycle, the "On" state of the pulse beam occupies 75% of the entire period, and an irradiation time of radiation is lengthened to provide a stronger stimulation effect. This setting may be applied to a patient in whom disease progression is advanced or in a situation requiring a high treatment intensity, but precise control according to individual characteristics of the patient is essential because a risk of tissue damage may increase due to the high irradiation ratio.

In the 25% duty cycle, the "On" state of the pulse beam corresponds to 25% of the entire period, and provides relatively low radiation exposure. This is a setting capable of promoting a long-term treatment effect while minimizing tissue damage, and is suitable for a sensitive patient or preventive treatment at an initial stage.

The pulse beam control system precisely controls a tube voltage and a tube current under each duty cycle condition to optimize energy intensity and an irradiation amount of radiation. The pulse beam control system finely adjusts an On/Off period of the pulse beam to control a pulse width, a period, and an irradiation frequency, so that customized setting is possible according to a biological response mechanism and a treatment effect. The pulse beam is irradiated in series or in parallel through multiple ports, and provides an energy distribution and an irradiation angle optimized for each patient site.

Through duty cycle control, a maximum treatment effect may be achieved with a minimum radiation dose. This enables patient-customized treatment and secures both safety and efficiency. This technology may be applied not only to degenerative neurological diseases such as Alzheimer's disease and Parkinson's disease, but also to treatment requiring inflammatory disease treatment and tissue regeneration. Each duty cycle condition is optimized to control various biological responses, such as inflammation suppression, tissue repair, and neural regeneration.

FIG. 6 visually explains a core control capability of the pulse beam control technology, and emphasizes, through this, the possibility of a radiation protocol that maximizes a treatment effect.

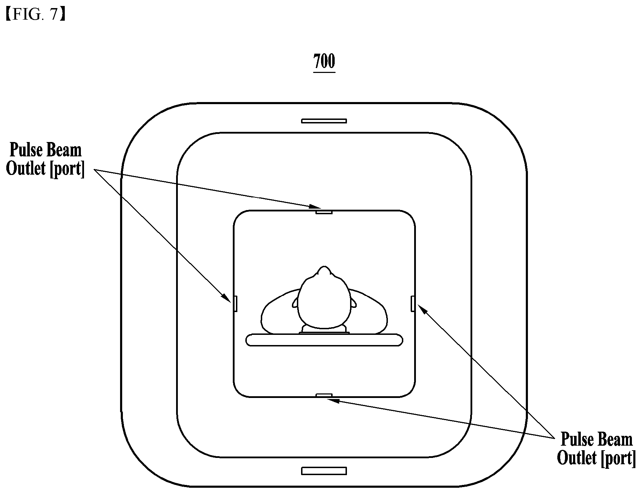

FIG. 7 is a diagram 700 illustrating application fields of the present invention.

FIG. 7 visually illustrates an application example in which radiation is precisely irradiated using a multi-gantry structure designed based on a pulsed beam (PKLDR, Pulsed Kilovoltage Low-Dose Radiation) technology.

FIG. 7 shows a method of providing patient-customized treatment by irradiating radiation optimized for a treatment target site using various pulse beam outlets. The system is designed to precisely control an irradiation angle and energy intensity of radiation and to maximize a treatment effect on target tissue while minimizing damage to surrounding tissue.

In FIG. 7, four pulse beam outlets are arranged, and the pulse beam outlets are designed to irradiate radiation around a patient.

Each outlet is independently controllable, and adjusts intensity and direction of radiation according to a location, size, and disease condition of tissue to be treated. The outlets cooperate with each other to irradiate radiation at multiple angles, and are particularly effective when targeting an atypical lesion or deep tissue.

The patient is positioned at a center of the gantry structure and fixed in a stable posture. The system adjusts a direction and energy of radiation in consideration of a body structure and pathological characteristics of the patient. The head and body of the patient are designed to be accurately positioned within a radiation irradiation area, thereby enabling treatment of sensitive sites such as the brain and neural tissue.

The multi-gantry optimizes a position so that each pulse beam outlet can accurately irradiate radiation to a specific site of the patient.

The system of FIG. 7 maintains an irradiation range uniformly and may irradiate radiation to various sites of the body of the patient simultaneously or sequentially.

Each pulse beam outlet independently adjusts a tube voltage and a tube current to optimize an amount and energy of radiation. A pulse width, an irradiation period, and a duration of radiation are precisely controlled to achieve a specific treatment target.

A radiation dose is checked during irradiation through a real-time dose monitoring system and is immediately adjusted when necessary. A multi-port design irradiates radiation from multiple directions so that radiation is concentrated on target tissue. For example, when treating a specific lesion site of the brain, the four outlets irradiate radiation from different angles to concentrate radiation on the lesion site and minimize exposure of surrounding normal tissue.

The system of FIG. 7 sets a radiation irradiation plan based on medical images such as MRI and CT data of the patient. The system analyzes a body structure, a lesion location, and tissue sensitivity of the patient, and individually optimizes irradiation conditions of the pulse beam, including a radiation dose, an irradiation angle, and an irradiation time.

The pulse beam control system is effective in suppressing neuroinflammation and reducing pathological markers, such as Aβ plaques and tau proteins, in neurodegenerative diseases such as Alzheimer's disease and Parkinson's disease.

Precise treatment of sensitive brain tissue is possible, and damage to surrounding tissue is minimized by concentrating radiation on a lesion site. In addition to brain diseases, the pulse beam control system may be used for various diseases such as arthritis, inflammatory diseases, and cancer treatment. Low-dose radiation is used to regulate an inflammatory response and promote tissue regeneration.

A maximum treatment effect can be achieved within a short time through the multi-gantry structure and a real-time control system. A treatment experience of the patient is improved, and side effects caused by radiation exposure are minimized.

FIG. 7 visually explains a method in which the pulse beam control system precisely performs radiation therapy using the multi-gantry structure.

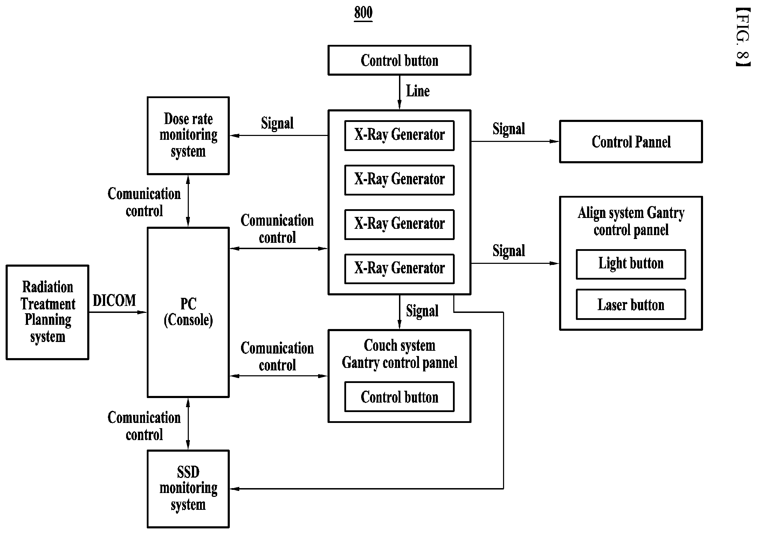

FIG. 8 is a diagram 800 illustrating an operation system diagram according to an embodiment.

FIG. 8 shows in detail an operation system diagram of a system implementing a pulsed beam (PKLDR, Pulsed Kilovoltage Low-Dose Radiation) technology.

FIG. 8 visually explains a structure of a multiple control system designed for various medical applications including treatment of a degenerative brain disease, and signal flows among respective components.

A Radiation Treatment Planning System establishes a radiation treatment plan, including a treatment area, irradiation intensity, and an irradiation angle, based on medical images such as MRI and CT of a patient.

The Radiation Treatment Planning System transmits data in a DICOM format to a PC (console), and the PC controls an X-ray Generator and other components based on the data.

The PC is a central control device of the present system, and is responsible for communication with and control of all components. The PC receives DICOM data and transmits and receives signals to and from main components, such as the X-ray Generator, a Dose Rate Monitoring System, an SSD Monitoring System, and a Couch System. The PC controls, in real time, a tube voltage, a tube current, a pulse width, an irradiation period, an irradiation timing, and the like, thereby enabling patient-customized treatment.

The Dose Rate Monitoring System measures, in real time, a radiation dose delivered to the patient during radiation irradiation. This data is transmitted to the PC, and radiation intensity and an irradiation time are immediately adjusted when necessary. This plays an important role in maximizing safety and a treatment effect of radiation.

The SSD Monitoring System measures a distance between the X-ray generator and the patient (Source-to-Surface Distance, SSD). Distance data is transmitted to the PC and used to optimize a radiation irradiation angle and intensity. Through this, the SSD Monitoring System ensures that radiation accurately reaches target tissue.

The X-ray Generator generates radiation based on signals transmitted from the PC. Each generator may independently adjust a tube voltage, a tube current, and a pulse width, and operates in series or in parallel through multiple ports. The generator irradiates radiation in a multi-gantry structure, thereby enabling precise and efficient treatment.

The Couch System and a Gantry Control Panel adjust a position of the patient and stably fix the patient so that radiation accurately reaches target tissue. The Gantry Control Panel accurately adjusts a treatment position of the patient, and a Light Button allows a treatment area to be visually confirmed.

A Control Panel integrally manages signals of the X-ray Generator and the Gantry Control Panel, and supervises an overall radiation irradiation process. This allows medical staff to easily operate the system.

Main Power supplies power to the entire system and maintains power signals so that each component can operate smoothly.

The system of FIG. 8 covers an entire process from establishment of a radiation treatment plan to radiation irradiation.

A treatment plan generated by the Radiation Treatment Planning System is transmitted to the PC, and the PC integrally controls all components based on the data. The X-ray Generator generates radiation according to commands received from the PC, and the Dose Rate Monitoring System and the SSD Monitoring System provide real-time data to detect a radiation dose and a distance.

The Couch System and the Gantry Control Panel optimize a position of the patient and an irradiation angle, and the Control Panel monitors an operation state of the entire system.

The system diagram of FIG. 8 may provide high precision and safety in various medical applications including treatment of a degenerative brain disease.

Integrated signal flows among the respective components enable patient-customized treatment, achieve a maximum treatment effect with minimum radiation, and minimize side effects caused by radiation exposure through multi-port control and real-time data feedback.

The present system may be expanded to various medical fields, such as cancer treatment and inflammatory diseases, as well as degenerative diseases.

For example, the Radiation Treatment Planning System of FIG. 8 corresponds to the defined pulse beam information setting unit.

The Radiation Treatment Planning System establishes a radiation treatment plan based on medical image data of the patient, and the treatment plan is converted into a DICOM format and transmitted to the PC (Console). This provides basic data for adjusting parameters of a radiation generator and enables patient-customized treatment.

The X-Ray Generator of FIG. 8 matches the defined beam controller and includes four independent radiation generators. According to parameters received from the PC (Console), the X-Ray Generator adjusts a tube voltage and a tube current to optimize an amount and energy of a radiation beam, and adjusts, in real time, a radiation irradiation period, a pulse width, and an irradiation time. Through this, the X-Ray Generator precisely controls a direction and intensity of radiation to optimize treatment of a lesion site and minimize an effect on surrounding normal tissue.

The Dose Rate Monitoring System of FIG. 8 corresponds to the defined dose monitoring unit, and measures, in real time, a radiation dose delivered to the patient during treatment. The measured data is transmitted to the PC (Console), so that an excessive or insufficient state is checked and immediate adjustment is possible. This continuously monitors radiation exposure, thereby ensuring safety of the patient and maximizing treatment efficiency.

The PC (Console) communicates with the radiation generator, the dose monitoring system, and the SSD monitoring system based on treatment planning data received from the Radiation Treatment Planning System, and integrally manages a treatment process.

The PC (Console) collects, processes, and stores all real-time data, and adjusts radiation parameters when necessary to immediately resolve an error that may occur during the treatment process. This acts as a central hub that improves stability and efficiency of treatment, and enables integrated operation of the entire system.

The PC (Console) operates as the defined controller together with the data management unit, coordinates communication among the respective components, and stores, in real time, data generated during the radiation treatment process. Based on the collected data, the PC (Console) controls a tube voltage, a tube current, a pulse width, and the like of the radiation generator. This is responsible for overall control and management of treatment, and maximizes stability of the system and a treatment effect.

The SSD Monitoring System of FIG. 8 measures, in real time, a distance (SSD) between the patient and the radiation generator to maintain radiation irradiation accuracy. The measured data is transmitted to the PC (Console) to adjust a radiation irradiation position and intensity, and immediately detects movement of the patient during treatment. Through this, accuracy of radiation irradiation is ensured, and unnecessary radiation exposure is prevented.

The Couch System and the Gantry Control Panel of FIG. 8 are configured as devices for fixing a position of the patient and controlling radiation irradiation. The Couch System and the Gantry Control Panel stably fix the patient, adjust a radiation irradiation angle and direction, and improve precision of treatment. A Light Button and a Laser Button confirm and determine an accurate position of the patient in a treatment preparation stage. During a radiation irradiation process, movement of the patient is minimized, and radiation is accurately irradiated to a treatment target site.

A treatment plan generated by the Radiation Treatment Planning System is transmitted to the PC (Console) in a DICOM format.

The PC (Console) analyzes data, communicates with the radiation generator, the dose monitoring system, and the SSD monitoring system, and integrally manages a treatment process. The X-Ray Generator irradiates radiation according to set parameters, and the Dose Rate Monitoring System and the SSD Monitoring System collect data in real time to maintain accuracy of the treatment process. The Couch System and the Gantry Control Panel maintain an accurate position of the patient and optimize a radiation irradiation direction and angle.

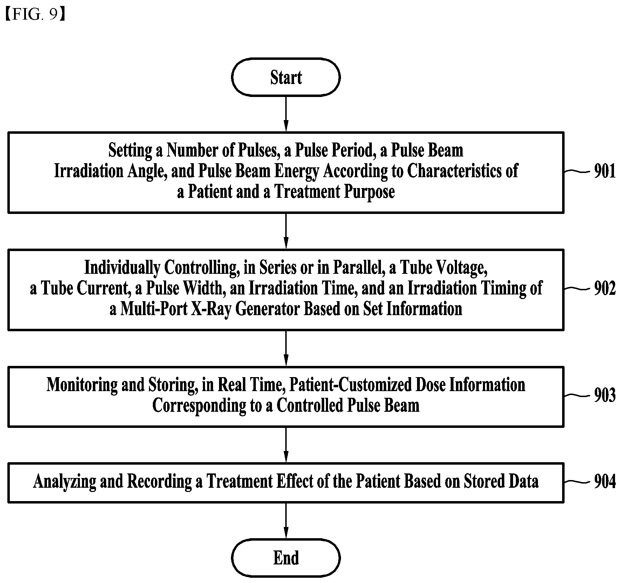

FIG. 9 is a diagram illustrating an operation method of a pulse beam control system for treating a degenerative brain disease according to an embodiment.

FIG. 9 is a diagram stepwise illustrating an operation method of a pulse beam control system (PKLDR, Pulsed Kilovoltage Low-Dose Radiation) for treating a degenerative brain disease, and specifically illustrates, in detail, a treatment process including planning, execution, monitoring, and evaluation of radiation treatment. This drawing emphasizes precision and effectiveness of radiation treatment through linkage with components defined in the claims.

A first step 901 according to an embodiment is a process of setting pulse beam parameters based on pathological characteristics and a treatment target of a patient to be treated. For this, a number of pulses, a pulse period, a pulse beam irradiation angle, and pulse beam energy are set according to characteristics of the patient and a treatment purpose. This is performed through a Radiation Treatment Planning System.

Medical image data such as MRI and CT are analyzed to identify a location and size of a lesion site and a state of surrounding tissue. Based thereon, a radiation irradiation area is determined.

The number of pulses means the number of times of radiation irradiation, and is optimized to maximize a biological response and minimize side effects.

The pulse period provides tissue recovery time between irradiations, and a pulse width controls a duration of radiation irradiation.

An irradiation angle and a direction are precisely set by using a multi-gantry structure, and concentrate radiation on the lesion site. The set pulse beam information is converted into a DICOM format and transmitted to the PC (Console).

A second step 902 is a process of generating and irradiating radiation based on data received at the PC (Console). Based on the set information, a tube voltage, a tube current, a pulse width, an irradiation time, and an irradiation timing of a multi-port X-ray generator are individually controlled in series or in parallel. An X-ray Generator performs a key role.

The system includes a multi-port X-ray generator, and each generator is independently controllable. The generator irradiates radiation by setting a radiation direction and intensity optimized for the lesion site.

Energy intensity and an irradiation amount of radiation are adjusted through a tube voltage and a tube current. A pulse width and a period are controlled according to a treatment target, and an irradiation timing is also precisely managed. By using a multi-gantry structure, radiation is irradiated to a lesion site from various angles, thereby minimizing radiation exposure to normal tissue.

A third step 903 is a process of monitoring, in real time, a radiation delivery state during treatment and storing data, and monitors and stores, in real time, patient-customized dose information corresponding to a controlled pulse beam. A Dose Rate Monitoring System measures, in real time, a radiation dose received by the patient during radiation irradiation.

Measured data is transmitted to the PC (Console), and an excessive or insufficient state is checked by comparing the measured data with a target dose.

An SSD Monitoring System measures, in real time, a distance between the radiation generator and the patient, and maintains radiation irradiation accuracy. All real-time data is stored and used for subsequent treatment planning and treatment effect analysis.

A last step 904 is a process of analyzing treatment data to evaluate and record a treatment effect, and analyzes and records a treatment effect of the patient based on the stored data. Based on the stored data, dose data and a biological response of the patient are comparatively evaluated, and reflected in improvement of a treatment protocol. Analysis results are recorded as treatment data and used for future treatment and system optimization.

The pulse beam control system enables patient-customized treatment by controlling a pulse width, a period, intensity, a direction, and the like in real time. Through real-time monitoring, radiation exposure is minimized, and a treatment effect is maximized.

For example, controlling the multi-port X-ray generator may include providing an irradiation angle and an energy distribution optimized for a body part of the patient using a multi-gantry structure.

In addition, according to the present invention, radiation may be uniformly distributed even to a complex lesion structure by using a multi-gantry structure, and patient-customized radiation treatment may be provided through precise control of pulse beam parameters.

Furthermore, long-term treatment effects may be analyzed and improved treatment strategies may be established by using stored treatment data, and radiation irradiation accuracy may be maintained by correcting minute movements of the patient in real time.

The pulse beam control system is applicable to various diseases, such as Alzheimer's disease, Parkinson's disease, tumor treatment, and inflammatory diseases. FIG. 9 stepwise explains how the pulse beam control system operates integrally throughout an entire radiation treatment process, and specifically illustrates a technical configuration for improving a treatment effect and safety. The present invention shows a possibility of becoming an innovative technology in various medical fields.

The apparatus described above may be implemented by hardware components, software components, and/or a combination of hardware components and software components. For example, the apparatus and components described in the embodiments may be implemented using one or more general-purpose computers or special-purpose computers, such as, for example, a processor, a controller, an arithmetic logic unit (ALU), a digital signal processor, a microcomputer, an FPA (field programmable array), a programmable logic unit (PLU), a microprocessor, or any other device capable of executing and responding to an instruction. A processing device may execute an operating system (OS) and one or more software applications executed on the operating system. In addition, the processing device may access, store, manipulate, process, and generate data in response to execution of software. For convenience of understanding, the processing device may be described as being used as one device, but a person having ordinary skill in the art will understand that the processing device may include a plurality of processing elements and/or a plurality of types of processing elements. For example, the processing device may include a plurality of processors, or one processor and one controller. In addition, other processing configurations, such as a parallel processor, are also possible.

Software may include a computer program, code, an instruction, or a combination of one or more thereof, and may configure the processing device to operate as desired or instruct the processing device independently or collectively. The software and/or data may be permanently or temporarily embodied in any type of machine, component, physical device, virtual equipment, computer storage medium or device, or a transmitted signal wave, in order to be interpreted by the processing device or to provide an instruction or data to the processing device. The software may be distributed over network-connected computer systems and stored or executed in a distributed manner. The software and data may be stored in one or more computer-readable recording media.

A method according to an embodiment may be implemented in the form of program instructions executable by various computer means and recorded in a computer-readable medium. The computer-readable medium may include program instructions, data files, data structures, and the like, alone or in combination. The program instructions recorded on the medium may be specially designed and configured for the embodiment, or may be known to and available to those skilled in computer software. Examples of the computer-readable recording medium include magnetic media such as a hard disk, a floppy disk, and a magnetic tape, optical media such as CD-ROM and DVD, magneto-optical media such as a floptical disk, and hardware devices specially configured to store and execute program instructions, such as ROM, RAM, and flash memory. Examples of the program instructions include not only machine language code, such as code generated by a compiler, but also high-level language code executable by a computer using an interpreter or the like. The above-described hardware device may be configured to operate as one or more software modules to perform operations of the embodiment, and vice versa.

Although the embodiments have been described above with reference to limited drawings, a person having ordinary skill in the art may make various modifications and variations from the above description. For example, appropriate results may be achieved even when the described technologies are performed in an order different from the described method, and/or components of the described system, structure, apparatus, circuit, and the like are coupled or combined in a form different from the described method, or replaced or substituted by other components or equivalents.

Therefore, other implementations, other embodiments, and equivalents to the claims also fall within the scope of the following claims.

## Claims

1. A pulse beam control system for treating a degenerative brain disease, the pulse beam control system being adapted for use with a carbon nanotube X-ray generator, the pulse beam control system comprising:
a pulse beam information setting unit configured to set pulse beam information including a number of pulses, a pulse period, a pulse beam irradiation angle, or pulse beam energy according to characteristics of a patient and a treatment purpose;
a beam controller configured to individually control, in series or in parallel, at least one of a tube voltage, a tube current, a pulse width, an irradiation time, or an irradiation timing of a multi-port X-ray generator based on the set pulse beam information;
a dose monitoring unit configured to monitor and store, in real time, patient-customized dose information corresponding to a generated pulse beam; and
a data management unit configured to manage and output treatment data of the patient based on the monitored data.

2. The pulse beam control system of claim 1, wherein the pulse beam information setting unit is configured to automatically calculate and set the pulse beam information based on treatment planning data received from an external system.

3. The pulse beam control system of claim 1, wherein the beam controller is configured to provide an irradiation angle and an energy distribution optimized for a body part of the patient through a multi-gantry structure.

4. The pulse beam control system of claim 1, wherein the dose monitoring unit is configured to collect data by communicating with a Dose Rate Monitoring System and an SSD Monitoring System to measure real-time dose information of the patient.

5. The pulse beam control system of claim 1, wherein the data management unit is configured to analyze dose data collected from the dose monitoring unit, evaluate a treatment effect for each patient, and control output of the evaluated treatment effect.

6. The pulse beam control system of claim 1, wherein the data management unit is configured to store treatment data and dose data of the patient and construct a database so that the stored treatment data and dose data are reusable as data required for subsequent treatment.

7. The pulse beam control system of claim 1, wherein the beam controller is configured to synchronize irradiation timings of pulse beams generated by respective X-ray generators and irradiate the pulse beams in parallel or in series.

8. The pulse beam control system of claim 1, wherein the dose monitoring unit is configured to use a feedback control algorithm to optimize a dose and an energy intensity of an irradiated pulse beam for each patient.

9. A pulse beam control method for treating a degenerative brain disease, the pulse beam control method comprising:
setting a number of pulses, a pulse period, a pulse beam irradiation angle, and pulse beam energy according to characteristics of a patient and a treatment purpose;
individually controlling, in series or in parallel, a tube voltage, a tube current, a pulse width, an irradiation time, and an irradiation timing of a multi-port X-ray generator based on the set information;
monitoring and storing, in real time, patient-customized dose information corresponding to a controlled pulse beam; and
analyzing and recording a treatment effect of the patient based on the stored data.

10. The pulse beam control method of claim 9, wherein setting the number of pulses, the pulse period, the pulse beam irradiation angle, and the pulse beam energy comprises automatically setting the number of pulses, the pulse period, the pulse beam irradiation angle, and the pulse beam energy based on patient data received from an external system.

11. The pulse beam control method of claim 9, wherein individually controlling the multi-port X-ray generator comprises providing an irradiation angle and an energy distribution optimized for a body part of the patient using a multi-gantry structure.
